# EUROPEAN PATENT APPLICATION

(11) **EP 4 653 534 A1**
(43) Date of publication of application: **26.11.2025**
(21) Application number: 24792875.7
(22) Date of filing: 26.03.2024
(51) Int. Cl.: C12N 15/77, C12N 9/12, C12P 13/22

(54) **MICROORGANISM FOR PRODUCING L-TRYPTOPHAN AND METHOD FOR PRODUCING L-TRYPTOPHAN USING SAME**

(30) Priority: 19.04.2023 KR 20230051258
(71) Applicant: CJ CheilJedang Corporation, Seoul 04560 (KR)
(72) Inventor: PARK, Seul-Gi, Seoul 04560 (KR); JUNG, Moo Young, Seoul 04560 (KR); PARK, Soe-hee, Seoul 04560 (KR); LEE, Sumin, Seoul 04560 (KR)
(74) Representative: Meissner Bolte Partnerschaft mbB
(86) International application number: PCT/KR2024/003775
(87) International publication number: WO 2024/219688

(57) **Abstract**

The present disclosure relates to a microorganism of the genus *Corynebacterium* having an L-tryptophan-producing ability, into which pyruvate, phosphate dikinase derived from *Komagataeibacter xylinus* or a polynucleotide encoding the same is introduced; a method for producing L-tryptophan, comprising culturing the microorganism in a medium; a composition for producing L-tryptophan, comprising the microorganism, a culture product of the microorganism, a fermented product of the microorganism, or a combination of two or more thereof; and the use of the microorganism for the production of L-tryptophan.

## Description

### [Technical Field]

The present disclosure relates to a microorganism of the genus *Corynebacterium* having an L-tryptophan-producing ability, into which pyruvate, phosphate dikinase derived from *Komagataeibacter xylinus* or a polynucleotide encoding the same is introduced; a method for producing L-tryptophan, including culturing the microorganism in a medium; a composition for producing L-tryptophan, including the microorganism, a culture product of the microorganism, a fermented product of the microorganism, or a combination of two or more thereof; and the use of the microorganism for the production of L-tryptophan.

### [Background Art]

A variety of studies on the production of target substances (e.g., amino acids) in microorganisms have been directed to eco-friendly and safe production methods, among which studies for producing target substances in large quantities in microorganisms of the genus *Corynebacterium* have been continuously conducted. A microorganism of the genus *Corynebacterium* (*Corynebacterium* sp.), especially *Corynebacterium glutamicum,* is a gram-positive microorganism that is often used to produce L-amino acids and other useful substances. For the production of L-amino acids and other useful substances, various studies for developing microorganisms with high-efficiency production and technologies for fermentation processes are underway.

L-tryptophan, which is an essential amino acid, has been widely used as a raw material for pharmaceutical products, such as feed additives, infusion solutions, *etc.,* and a health food ingredient, *etc.* As such, L-tryptophan can be produced by a chemical synthesis method, an enzyme reaction method, a fermentation method, *etc.,* but the direct fermentation method using a microorganism is mostly used at present.

Microorganisms have an aromatic biosynthetic pathway, in which phosphoenol pyruvate (PEP), which is an intermediate in glycolysis, and erythrose-4-phosphate (E4P), which is a product of the pentose phosphate pathway, initiate polymerization by 3-deoxy-D-arabinoheptulosonate 7-phosphate (DAHP) synthase (EC 2.5.1.54), during the biosynthesis of L-tryptophan. According to the previous studies, it was demonstrated by intracellular quantitative analysis that the highest level of energy is required for biosynthesis of tryptophan from among 20 amino acids (Proc. Natl. Acad. Sci. USA, (2002) V 99, pp 3695-3700).

Thus, in order to stably supply E4P, the method of increasing the biosynthesis by enhancing the expression of *tktA* gene (NCBI gene ID: 12931960), which encodes transketolase (EC 2.2.1.1), has been studied (Current Opinion in Biotechnology, (2009) V20, pp 651-658). In addition, studies on reducing the use of ATP, which is a high energy substance, are underway in order to maintain the intracellular energy level (FEMS Microbiol Lett, (2009) V297, pp 217-224).

However, gluconeogenesis is generally known to have very low activity in nutrient-rich culture environments (J Bacteriol. 2013 Sep, 195(18), 4283-4296.; Nature Communications volume 8, Article number: 14316, 2017). Moreover, there is a problem that the amount of phosphoenol pyruvate, a tryptophan precursor, is decreased due to the production of by-products such as acetic acid during the production process of L-tryptophan, thereby reducing the production of L-tryptophan.

Therefore, research on effectively increasing L-tryptophan production ability is still necessary.

### [Disclosure]

### [Technical Problem]

The present inventors have confirmed that the L-tryptophan-producing ability was increased in the microorganism of the genus *Corynebacterium,* into which pyruvate, phosphate dikinase derived from *Komagataeibacter xylinus* or a polynucleotide encoding the same is introduced, thereby completing the present disclosure.

### [Technical Solution]

It is one object of the present disclosure to provide a microorganism of the genus *Corynebacterium* having an L-tryptophan-producing ability, into which pyruvate, phosphate dikinase derived from *Komagataeibacter xylinus* or a polynucleotide encoding the same is introduced.

In one embodiment, the pyruvate, phosphate dikinase derived from *Komagataeibacter xylinus* may include an amino acid sequence of SEQ ID NO: 1
In another embodiment, the pyruvate, phosphate dikinase derived from *Komagataeibacter xylinus* may be encoded by a *ppdK* gene.

As the microorganism according to any one of the above-described embodiments, the microorganism of the genus *Corynebacterium* may be *Corynebacterium glutamicum.*

As the microorganism according to any one of the above-described embodiments, the microorganism of the genus *Corynebacterium* may have an increased L-tryptophan-producing ability compared to a non-modified microorganism.

It is another object of the present disclosure to provide a method for producing L-tryptophan, including culturing a microorganism of the genus *Corynebacterium* having an L-tryptophan-producing ability, into which pyruvate, phosphate dikinase derived from *Komagataeibacter xylinus* or a polynucleotide encoding the same is introduced, in a medium

In one embodiment, the method may further include recovering L-tryptophan from the cultured microorganism, a culture product of the microorganism, a fermented product of the microorganism, or the culture medium.

It is still another object of the present disclosure to provide a composition for producing L-tryptophan, including: a microorganism of the genus *Corynebacterium* having an L-tryptophan-producing ability, into which pyruvate, phosphate dikinase derived from *Komagataeibacter xylinus* or a polynucleotide encoding the same is introduced; a culture product of the microorganism; a fermented product of the microorganism; or a combination of two or more thereof.

It is yet another object of the present disclosure to provide the use of a composition, which includes a microorganism of the genus *Corynebacterium* having an L-tryptophan-producing ability, into which pyruvate, phosphate dikinase derived from *Komagataeibacter xylinus* or a polynucleotide encoding the same is introduced; a culture product of the microorganism; a fermented product of the microorganism; or a combination of two or more thereof, for the production of L-tryptophan

### [Advantageous Effects]

The microorganism of the genus *Corynebacterium* having an L-tryptophan-producing ability, into which pyruvate, phosphate dikinase derived from *Komagataeibacter xylinus* or a polynucleotide encoding the same is introduced, provided in the present disclosure can produce L-tryptophan with high yields and thus can be useful in the industrial production of L-tryptophan.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety and the level of technical field to which the present disclosure belongs and the contents of the present disclosure will be described more clearly.

### Definitions

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or".

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." may be used to distinguish similar constitutions. When the terms are used in reference to steps of a method, use, or assay, these terms do not mean that the steps are performed continually or sequentially. For example, there may be no time interval between these steps, or they may be performed concurrently, or may be performed sequentially, randomly or in reverse order with several seconds, several minutes, several hours, several days, or several months apart.

As used herein, the term, "consisting of" means that the total ratio of specific features, steps, ingredients or other component(s) recited after the term is 100%. The features, steps, ingredients or other component(s) that are recited after the term "consisting of" may be essential or mandatory. For example, in addition to the features, steps, ingredients or other component(s) recited after the term "consisting of", any other features, steps, ingredients or other component(s), or non-essential features, steps, ingredients or other component(s) may be excluded.

As used herein, the term "consisting essentially of" may mean that when the features, steps, ingredients or other component(s) of the object claimed herein are not substantially affected by the presence of one or more unspecified features, steps, ingredients or other component(s), the one or more unspecified features, steps, ingredients or other component(s) may be present.

As used herein, the term "comprising/including" means the presence of features, steps, ingredients or other component(s) recited after the term, and does not exclude the presence or addition of one or more features, steps, ingredients or other component(s). The features, steps, ingredients or other component(s) recited after the term "comprising/including" herein may be essential or mandatory. However, in some embodiments, the term may further include any other or non-essential features, steps, ingredients or other component(s).

### Proteins, Polypeptides

As used herein, the term "protein" or "polypeptide" refers to a polymer or oligomer of consecutive amino acid residues. In the present disclosure, the "polypeptide", "protein", and "peptide" may be used interchangeably with each other.

In some cases, the term "an amino acid sequence exhibiting activity" may mean the "polypeptide", "protein", or "peptide", and when the "polypeptide", "protein", "peptide" or "amino acid sequence exhibiting activity" has a catalytic activity, it may be referred to as an "enzyme".

As used herein, the term "mature polypeptide" or "mature protein" means a polypeptide or protein in a form without a signal sequence or pro-peptide sequence. A mature polypeptide or mature protein may be a functional form of a polypeptide or protein. The mature polypeptide or mature protein may refer to a polypeptide in a final form after translation; and/or post-translational modification. Examples of the post-translational modification include N-terminal processing, C-terminal truncation, glycosylation, phosphorylation, leader sequence removal, *etc.,* but are not limited thereto.

In the present disclosure, unless indicated otherwise, the amino acid sequences are described in an N-terminal to C-terminal direction.

With respect to the amino acid sequences in the present disclosure, although it is described as a polypeptide or protein "comprising/including" an amino acid sequence described by a specific sequence number, a polypeptide or protein "consisting of" an amino acid sequence described by a specific sequence number, or a polypeptide or protein "having" an amino acid sequence described by a specific sequence number, it is apparent that any polypeptide or protein having an amino acid sequence in which part of the sequence(s) is deleted, modified, substituted, conservatively substituted or added may fall within the scope of the present disclosure if it has activity identical or corresponding to the polypeptide or protein consisting of the amino acid sequence of the corresponding sequence number. For example, polypeptides or proteins having sequence addition or deletion that do not alter the function of the protein, naturally occurring mutations, silent mutation thereof, or conservative substitutions within, or upstream or downstream (N-terminal or C-terminal) of the polypeptide or protein sequences may be included, as long as they have activity identical or corresponding to the activity of the polypeptide or protein.

For example, polypeptides or proteins that may be conjugated with a signal (or leader) sequence at the N-terminal involved in the translocation of proteins (polypeptides) co-translationally or post-translationally, or polypeptides or proteins that may be conjugated with another sequence or linker to identify, purify, or synthesize the polypeptides or proteins may also fall within the scope of the polypeptide or protein of the amino acid sequence described by the specific number.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chain (electrically charged amino acids) include arginine, lysine, histidine, glutamic acid, and aspartate; and amino acids having uncharged side chains (uncharged amino acid; also referred to as neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids may be classified according to their size into 5 groups, starting from the amino acid groups with a relatively small volume, i.e., glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto. Typically, conservative substitutions may have little or no effect on the activity of the polypeptide or protein.

### Gene, Polynucleotide

As used herein, the term "gene" means a polynucleotide that encodes a functional molecule and a polynucleotide including the regions upstream and downstream of the polynucleotide, or a functional RNA. In some embodiments, a gene may have a sequence (intron) inserted between each coding region (exon).

As used herein, the term "polynucleotide", "nucleic acid", or "nucleic acid molecule" which is a polymer of nucleotides composed of nucleotide monomers connected in a lengthy chain by a covalent bond, is a DNA (e.g., cDNA or genomic DNA) or RNA (e.g., mRNA) strand having at least a certain length. In the present disclosure, the "polynucleotide", "nucleic acid", or "nucleic acid molecule" may be used interchangeably with each other.

### Identity, Homology

As used herein, the term "identity" or "homology" refers to the degree of relatedness between two given amino acid sequences or nucleotide sequences, and may be expressed as a percentage. The terms "homology" and "identity" may often be used interchangeably with each other.

The sequence homology or identity of conserved polynucleotides or polypeptides may be determined by standard alignment algorithms and may be used together with a default gap penalty established by the program being used.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

Further, whether any two polynucleotide sequences have homology, similarity, or identity may be determined by comparing sequences via Southern hybridization experiments under defined stringent conditions, and the appropriate hybridization conditions to be defined may be determined by way of a method within the scope of the present disclosure, which is known to those skilled in the art (*e.g.,* J. Sambrook et al., Molecular Cloning, A Laboratory Manual*;* F. M. Ausubel et al., Current Protocols in Molecular Biology, John Wiley & Sons, Inc., New York), but is not limited thereto. Substantially, homologous or identical polynucleotide sequences are generally expected to hybridize to all or at least about 50%, 60%, 70%, 80% or 90% of the entire length of the sequences under stringent conditions.

As used herein, the "stringent conditions" refer to conditions which allow the specific hybridization between polynucleotides. Such conditions are disclosed in detail in the literature (see, Sambrook et al., supra, 9.50-9.51, 11.7-11.8). For example, the stringent conditions may include conditions under which polynucleotides having high homology or identity, i.e., polynucleotides having 60% or more, 70% or more, 75% or more, 80% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleotides have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the present disclosure may also include an isolated nucleic acid fragment complementary to the entire sequence as well as a base sequence substantially similar thereto.

For example, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (*e.g.,* Sambrook et al., supra).

### Nucleic Acid Construct, Vector, Transformation

As used herein, the term "nucleic acid construct" refers to a single- or doublestranded nucleic acid molecule, which includes one or more regulatory sequences, and which is artificially synthesized, or engineered to include a specific sequence in a manner that does not exist in nature, or isolated from nature.

As used herein, the term "vector" refers to a DNA construct for delivering a target polynucleotide into a suitable host or host cell. In oon example, the vector may contain the nucleotide sequence of a polynucleotide encoding the target polypeptide operably linked to a suitable expression regulatory region (expression regulatory sequence) so as to be able to express the target polypeptide in a suitable host cell, but is not limited thereto.

The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell (microorganism), the vector may replicate or function independently from the host genome, or may integrate into the genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pDC, pBR, pUC, pBluescriptll, pGEM, pTZ, pCL, and pET, *etc.* may be used. In one example, pDZ, pDC, pACYC177, pACYC184, pCL, pECCG117, pUC19, pBR322, pMW118, pCC1BAC vector, *etc.* may be used.

In one example, a target polynucleotide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming the insertion of the target polynucleotide, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a target polynucleotide into a host cell (microorganism) to alter the genetic characteristics of the host cell (microorganism). The transformed polynucleotide may be integrated into the chromosome of the host cell (microorganism) and located therein or located extrachromosomally. Further, the polynucleotide may include DNA and/or RNA. The polynucleotide may be introduced in a suitable form depending on the purpose of introduction. For example, the polynucleotide for expressing the target polypeptide may be introduced into the host cell (microorganism) in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell (microorganism) as it is and operably linked to sequences required for expression in the host cell, but is not limited thereto.

As used herein, the term "operably linked" refers to a constitution of placing a regulatory sequence in an appropriate position to regulate the expression of a coding sequence. Thus, the term "operably linked" includes an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, it may mean that the polynucleotide sequence is functionally linked to a promoter sequence that initiates and mediates transcription of the polynucleotide encoding the target polypeptide.

As used herein, the term "expression" includes any step involved in the production of a polypeptide, e.g., transcription, post-transcriptional modification, translation, post-translational modification, and secretion, *etc.,* but is not limited thereto.

As used herein, the term "expression vector" refers to a linear or circular nucleic acid molecule including a target polynucleotide sequence and an operably linked regulatory sequence for expression thereof. For example, it may include the nucleotide sequence of a polynucleotide encoding the target polypeptide operably linked to a suitable expression regulatory region (expression regulatory sequence) so as to be able to express the target polypeptide in a suitable host cell.

As used herein, the term "regulatory sequence" refers to a polynucleotide sequence necessary for the expression of a target polynucleotide sequence. Each regulatory sequence may be native (derived from the same origin) or foreign (derived from different genes) to the coding sequence, or may be a mutant sequence thereof or other artificial sequences. Examples of the regulatory sequence may include a leader sequence, a polyadenylation sequence, a pro-peptide sequence, a promoter, a signal peptide sequence, an operator sequence, a sequence encoding a ribosome-binding site, and a sequence for regulating the termination of transcription and translation. The minimum units of the regulatory sequence may include a promoter, and a sequence for terminating transcription and translation.

As used herein, the term "genetic recombination" refers to a natural or artificial process in which the elements constituting the genes, such as DNA or RNA, are altered from their original sequence during disassembly and reassembly.

As used herein, the term "recombinant gene" refers to a gene with a new genomic constitution that develops as a result of genetic recombination, *e.g.,* chemical synthesis or genetic engineering technology, *etc.* As used herein, the terms "recombinant gene", "recombinant DNA" and "recombinant polynucleotide" may be used interchangeably with each other. In one example, the recombinant gene may include an artificial combination of nucleic acid fragments, such as regulatory sequences, that are not found in nature.

As used herein, the term "recombinant protein" refers to a protein produced as a result of genetic recombination.

### Microorganism

As used herein, the term "microorganism (or strain)" includes all wild-type microorganisms, or prokaryotic or eukaryotic microorganisms in which natural or artificial genetic modifications occur, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product. In the present disclosure, the "microorganism", "strain", "host" and "host cell" may be used interchangeably with each other.

As used herein, the term "recombinant microorganism" refers to a microorganism that has been genetically modified and exhibits a different genotype and/or phenotype compared to a naturally occurring microorganism (*e.g*., when the genetic modifications have an effect on a coding nucleic acid sequence of a microorganism), and may include progeny or all potential progeny of the microorganism. As used herein, the terms "recombinant microorganism", "genetically modified microorganism", "recombinant host cell", "recombinant cell" and "recombinant strain" may be used interchangeably with each other. The recombinant microorganism, for example, may express genes that are not found in the native (non-recombinant) form, or may not express genes that are expressed in its native form, or may express native genes in a manner different from that expressed in its native form.

For example, the microorganism of the present disclosure may be a microorganism (*e.g*., recombinant microorganism) into which pyruvate, phosphate dikinase or a polynucleotide encoding the same is introduced, but is not limited thereto.

As used herein, the term "microorganism having an L-tryptophan-producing ability", which is a microorganism capable of producing L-tryptophan in an organism, may include all microorganisms imparted with an L-tryptophan-producing ability which did not endogenously have an L-tryptophan-producing ability, or microorganisms that endogenously have an L-tryptophan-producing ability. The L-tryptophan-producing ability may be imparted or enhanced by improvement of species.

As used herein, the term "non-modified microorganism (strain)" does not exclude a microorganism (strain) containing a mutation that may occur naturally, and may refer to a wild-type microorganism (strain) or natural-type microorganism (strain) itself, or a microorganism (strain) before its trait is altered due to genetic modification caused by natural or artificial factors. As used herein, the "non-modified microorganism (strain)" may be used interchangeably with "microorganism (strain) before modification", "non-mutant microorganism (strain)", "parent microorganism", "parent strain", "wild-type microorganism (strain)", "reference microorganism (strain)", or "standard microorganism (strain)". In the present disclosure, the term may refer to a microorganism (strain), into which pyruvate, phosphate dikinase or a polynucleotide encoding the same is not introduced, or a microorganism (strain) before introduction thereof, but is not limited thereto. Additionally, the non-modified microorganism in the present disclosure may be a microorganism not including the polypeptide composed of SEQ ID NO: 1 or the polynucleotide composed of SEQ ID NO: 2, but is not limited thereto.

### Increase of Protein (Polypeptide) Activity

As used herein, the term "increase" of protein (polypeptide) activity means that the activity of a protein (polypeptide) is increased in a host cell (microorganism), as compared with the endogenous activity thereof. The increase may be used interchangeably with terms such as activation, up-regulation, overexpression, enhancement, *etc*. The host cell (microorganism) may be a prokaryotic or eukaryotic microorganism.

The increase of protein (polypeptide) activity may include both cases in which a protein (polypeptide) activity not endogenously possessed by a host cell (microorganism) is exhibited, or a protein (polypeptide) activity is enhanced compared to the endogenous activity or the activity before modification.

For example, the description "exhibiting a protein (polypeptide) activity not endogenously possessed" or exhibiting an improved protein(polypeptide) activity may be caused by the "introduction of a protein (polypeptide)", but is not limited thereto.

As used herein, the term "introduction" of protein (polypeptide) means that a gene not originally possessed by a microorganism is expressed in the microorganism, and thus the microorganism exhibits the activity of a particular protein, or the activity of a polypeptide is enhanced, increased or improved compared to the endogenous activity of the corresponding protein or the activity before modification. For example, it may be caused by the introduction of a gene encoding the protein (polypeptide) into a host cell (microorganism). For example, it may be a case in which a polynucleotide encoding a particular protein (polypeptide) is introduced into the chromosome of a host cell (microorganism), or a vector containing a polynucleotide encoding a particular protein (polypeptide) is introduced into a host cell (microorganism), thereby exhibiting its activity or improving the activity.

The "endogenous activity" refers to the activity of a particular protein (polypeptide) originally possessed by a host cell (microorganism) before transformation or a non-modified host cell (microorganism) when a trait is altered by genetic variation due to a natural or artificial factor. The endogenous activity may also be interchangeably used with "activity before modification".

The increase in the activity of a protein (polypeptide) as compared to the endogenous activity means that the activity and/or concentration (expression level) of the protein (polypeptide) of a host cell (microorganism) is improved, as compared with the activity and/or concentration (expression level) of the polypeptide originally possessed by a host cell (microorganism) before transformation or a non-modified host cell (microorganism).

In one example, the increase may mean that the activity of a corresponding protein (polypeptide) not originally exhibited is exhibited, or the activity or concentration thereof is increased generally by about 1% or more, about 10% or more, about 25% or more, about 50% or more, about 75% or more, about 100% or more, about 150% or more, about 200% or more, about 300% or more, about 400% or more, or about 500% or more, maximum about 1000% or about 2000% or more, based on the activity or concentration of a host cell (microorganism) before transformation or a non-modified host cell (microorganism), but is not limited thereto.

The enhancement of the activity of the protein (polypeptide) may be achieved by introducing a foreign protein (polypeptide) or increasing the activity of an endogenous protein (polypeptide). Whether or not the activity of the protein (polypeptide) is enhanced may be confirmed from the activity level of the corresponding protein (polypeptide), expression level thereof, or the increase in the amount of products produced from the corresponding protein (polypeptide).

The enhancement of the activity of the protein (polypeptide) may be applied by various methods well known in the art, and is not limited as long as it can enhance the activity of a target protein (polypeptide) compared to that of a host cell (microorganism) before modification. Specifically, genetic engineering and/or protein engineering well known to those skilled in the art, which is a common method of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the enhancement of the protein (polypeptide) of the present disclosure may be achieved by:
1) increasing the intracellular copy number of a polynucleotide encoding the protein (polypeptide);
2) modifying the expression regulatory region of a gene encoding the protein (polypeptide) on the chromosome *(e.g.,* inducing a modification within the expression regulatory region, replacing it with a sequence having a stronger activity, or inserting a sequence having a stronger activity);
3) modifying the nucleotide sequence encoding the initiation codon or 5'-UTR of the gene transcript encoding the protein (polypeptide);
4) modifying the amino acid sequence of the protein (polypeptide) such that the activity of the protein (polypeptide) is enhanced;
5) modifying the polynucleotide sequence encoding the protein (polypeptide) such that the activity of the protein (polypeptide) is enhanced *(e.g.,* modifying the polynucleotide sequence of the coding gene to encode a protein (polypeptide) that has been modified to enhance the activity of the protein (polypeptide));
6) introducing a foreign protein (polypeptide) exhibiting the activity of the protein (polypeptide) or a foreign polynucleotide encoding the same;
7) codon-optimization of a polynucleotide encoding the protein (polypeptide);
8) analyzing the tertiary structure of the protein (polypeptide) and thereby selecting and modifying the exposed site, or chemically modifying the same; or
9) a combination of two or more selected from above 1 to 8), but is not particularly limited thereto.

For example,
The 1) method of increasing the intracellular copy number of a polynucleotide encoding the protein (polypeptide) may be achieved by introducing a vector containing a polynucleotide encoding the protein (polypeptide), which is operably linked to a suitable regulatory sequence, into a host cell (microorganism). Alternatively, the method may be achieved by introducing one copy or two or more copies of polynucleotides encoding the protein (polypeptide), which is operably linked to a suitable regulatory sequence, into the chromosome of the host cell (microorganism). The introduction into the chromosome may be performed by introducing a vector, which is able to insert the polynucleotide into the chromosome of the host cell (microorganism), into the host cell (microorganism), but is not limited thereto. The vector is as described above. The regulatory sequence may be native (derived from the same origin) or foreign (derived from different genes) to the coding polynucleotide sequences, or may be a mutant sequence thereof or other artificial sequences, and may induce the expression of the polynucleotide in the host cell (microorganism).

The 2) method of replacing the expression regulatory region (or expression regulatory sequence) of a gene encoding the protein (polypeptide) on the chromosome with a sequence having a strong activity may be achieved, for example, by inducing a modification on the sequence through deletion, insertion, substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc*. In one example, the method may include replacing the original promoter with a strong promoter, but is not limited thereto.

Examples of the known strong promoter may include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene encoding the protein (polypeptide) may be achieved, for example, by modifying the nucleotide sequence to encode another initiation codon having a higher expression rate of the protein (polypeptide) compared to the endogenous initiation codon, or a RBS (ribosome binding site) sequence having a higher expression rate of the protein (polypeptide) compared to the endogenous RBS sequence, but is not limited thereto.

The 4) and 5) methods of modifying the amino acid sequence or the polynucleotide sequence of the protein (polypeptide) may be achieved by inducing a modification on the sequence through deletion, insertion, or substitution of the amino acid sequence of the protein (polypeptide) or the polynucleotide sequence encoding the protein (polypeptide), or a combination thereof to enhance the activity of the protein (polypeptide), or by replacing the sequence with an amino acid sequence or a polynucleotide sequence modified to increase the activity, but are not limited thereto. The replacement may be, for example, performed by inserting the polynucleotide into the chromosome by homologous recombination, but is not limited thereto.

The 6) method of introducing a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide may be used without limitation regardless of its origin or sequence as long as it exhibits the same/similar activity to that of the protein (polypeptide). The introduction may be performed by those of ordinary skill in the art by appropriately selecting a transformation method known in the art, and the expression of the introduced polynucleotide in the host cell enables the production of the protein (polypeptide), thereby increasing its activity.

The 7) method of codon-optimization of the polynucleotide encoding the protein (polypeptide) may be achieved by codon-optimization of an endogenous polynucleotide to increase the transcription or translation within a host cell (microorganism), or by optimizing the codons thereof such that the optimized transcription and translation of the foreign polynucleotide can be achieved within the host cell (microorganism).

The 8) method of analyzing the tertiary structure of the protein (polypeptide) and thereby selecting and modifying the exposed site, or chemically modifying the same may be achieved, for example, by comparing the sequence information of the protein (polypeptide) to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such an increase of the protein (polypeptide) activity may mean that the activity or concentration of the corresponding protein (polypeptide) is increased relative to the activity or concentration of a protein (polypeptide) expressed in a wild-type or a host cell (microorganism) before modification, or that the amount of products produced from the corresponding protein (polypeptide) is increased, but is not limited thereto.

The modification of a part or all of the polynucleotide in the microorganism of the present disclosure may be achieved by (a) homologous recombination through a vector for chromosomal insertion in the microorganism or genome editing using an engineered nuclease (e.g., CRISPR-Cas9), and/or (b) being induced by light, such as ultraviolet rays and radiation, *etc.* and/or chemical treatments, but is not limited thereto.

### Cultivation

As used herein, the term "cultivation" means that microorganisms are grown under appropriately controlled environmental conditions. The cultivation process may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of microorganisms as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. For example, the microorganism of the present disclosure may be cultured under aerobic conditions in a conventional medium containing an appropriate carbon source, nitrogen source, phosphorus source, inorganic compounds, amino acids, and/or vitamins, *etc.*, while adjusting temperature, pH, *etc.* For example, the culture medium for the microorganisms of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc*.; amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.*, molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected into the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for 10 hours to 160 hours, but is not limited thereto.

As used herein, the term "culture product" means a culture solution, a concentrated culture solution, a dried product of a culture solution, a culture filtrate, a concentrated culture filtrate, or a dried product of a culture filtrate obtained by culturing a specific microorganism in a culture medium, and means that the culture solution may include the specific microorganism while the culture filtrate does not substantially include the specific microorganism (in particular, it substantially means excluding a specific microorganism isolated by filtration, *etc.,* but does not mean that the microorganism is completely excluded from the filtrate). The formulation of the culture product is not limited, and may be, for example, a liquid, emulsion, or solid.

As used herein, the term "fermentation" means that microorganisms are not putrefactive during the process of decomposing organic matter using their own enzymes. Fermentation reaction and decay reaction proceed by similar processes, but when decomposition produces useful substances, it is called fermentation, and when odorous or harmful substances are produced, it is called decay.

In the present disclosure, the method for obtaining a fermented product from the microorganism is not particularly limited, and may be obtained according to a method commonly used in the art or similar fields.

As used herein, the term "fermented product" may include not only the fermented material itself, but also all kinds of materials including fermented products produced from the microorganism, such as a material including the fermented microorganism; a fermented product produced from the fermented microorganism; a fermented product of a culture product; a concentrated fermented product; a dried product of the fermented product, a filtrate of the fermented product; a filtrate of the concentrated fermented product, a dried product of the filtrate of the fermented product, an extract of the fermented product, or a diluted solution of the fermented product, *etc.*

### Detailed Description of the Present Disclosure

Hereinafter, the embodiments of the present disclosure will be described in detail as follows:
One aspect of the present disclosure provides a microorganism of the genus *Corynebacterium* having an L-tryptophan-producing ability, into which pyruvate, phosphate dikinase derived from *Komagataeibacter xylinus* or a polynucleotide encoding the same is introduced.

As used herein, the term "pyruvate, phosphate dikinase (PPDK)" refers to an enzyme in the transferase family that catalyzes the reaction of [ATP + pyruvate + phosphate ↔ phosphoenolpyruvate (PEP) + diphosphate]. The pyruvate, phosphate dikinase of the present disclosure may be used interchangeably with PPDK. Specifically, the pyruvate, phosphate dikinase of the present disclosure may be a protein having the activity of pyruvate, phosphate dikinase encoded by the *ppdk* gene, but the type thereof is not particularly limited as long as the protein has activity corresponding to the activity of pyruvate, phosphate dikinase. The pyruvate, phosphate dikinase encoded by the *ppdk* gene is known in the art, and the amino acid and polynucleotide sequences of the pyruvate, phosphate dikinase can be obtained from a known database, NCBI's GenBank, *etc.,* but is not limited thereto.

In one example, the pyruvate, phosphate dikinase derived from *Komagataeibacter xylinus* may include the amino acid sequence of SEQ ID NO: 1, or an amino acid sequence having 60% or more homology or identity thereto, but is not limited as long as it has an activity of pyruvate, phosphate dikinase. Specifically, any protein having the amino acid sequence of SEQ ID NO: 1, in which part of the sequence is deleted, modified, substituted, or added, may fall within the scope of the pyruvate, phosphate dikinase as long as it exhibits an efficacy corresponding to that of the pyruvate, phosphate dikinase. Additionally, any protein which has or includes an amino acid sequence having at least 60%, 70%, 80%, 85%, 90%, 95%, 96%, 97%, 98%, or 99% or more homology or identity to SEQ ID NO: 1, consists of the amino acid sequence above, or essentially consists of the amino acid sequence above, and exhibits an efficacy corresponding to that of the pyruvate, phosphate dikinase may fall within the scope of the pyruvate, phosphate dikinase.

In addition, the sequences of the polynucleotide encoding the pyruvate, phosphate dikinase derived from *Komagataeibacter xylinus* having the amino acid sequence of SEQ ID NO: 1 or an amino acid sequence having 60% or more homology or identity may be obtained, for example, based on the codon information known in the art. In one example, the pyruvate, phosphate dikinase may be encoded by a polynucleotide which may have or include the nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more, or 99% or more homology or identity to the nucleotide sequence of SEQ ID NO: 2, consist of the nucleotide sequence above, or essentially consist of the nucleotide sequence above, but is not limited thereto. Further, the nucleotide sequence of SEQ ID NO: 2 can be obtained from a known database, NCBI's GenBank, *etc.,* but is not limited thereto

In the present disclosure, the polynucleotide (gene) including the nucleotide sequence of SEQ ID NO: 2 may be used interchangeably with the polynucleotide (gene) having the nucleotide sequence of SEQ ID NO: 2, the polynucleotide (gene) consisting of the nucleotide sequence of SEQ ID NO: 2, or *ppdk.*

The polynucleotide of the present disclosure may undergo various modifications in the coding region without changing the amino acid sequence of the pyruvate, phosphate dikinase of the present disclosure, due to codon degeneracy or in consideration of the codons preferred in an organism in which the pyruvate, phosphate dikinase of the present disclosure is to be expressed. Therefore, based on codon degeneracy, it is apparent that polynucleotides which may be translated into polypeptides consisting of the amino acid sequence of the pyruvate, phosphate dikinase of the present disclosure or polypeptides having a homology or identity thereto may also be included. For example, the polynucleotide of the present disclosure may be SEQ ID NO: 2, or a degenerated sequence thereof.

In another example, the polynucleotide of the present disclosure may have or include a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, or may consist of or essentially consist of include a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, but is not limited thereto.

Additionally, the polynucleotide of the present disclosure may include a probe that may be prepared from a known gene sequence, for example, any sequence which may hybridize with a sequence complementary to all or part of the polynucleotide sequence of the present disclosure under stringent conditions to encode the pyruvate, phosphate dikinase of the present disclosure without limitation.

For the purpose of the present disclosure, the microorganism of the present disclosure may include all microorganisms capable of producing the desired L-tryptophan by introducing the pyruvate, phosphate dikinase or a polynucleotide encoding the same. For example, the microorganism of the present disclosure is characterized in that the activity of pyruvate, phosphate dikinase is introduced, thereby increasing the L-tryptophan-producing ability, and it may be a genetically modified microorganism or a recombinant microorganism, but is not limited thereto. Specifically, the recombinant strain having an increased L-tryptophan-producing ability may be a microorganism having an increased L-tryptophan-producing ability compared to a natural wild-type microorganism, or a non-modified microorganism having an endogenous activity of pyruvate, phosphate dikinase or having no endogenous activity of pyruvate, phosphate dikinase, but is not limited thereto.

In one example, the microorganism having an L-tryptophan-producing ability, which is a prokaryotic or eukaryotic microbial strain capable of producing L-tryptophan in an organism, may include all microorganisms that endogenously have an L-tryptophan-producing ability, or microorganisms, in which an L-tryptophan-producing ability has been imparted to a parent strain having no L-tryptophan-producing ability by the activity of pyruvate, phosphate dikinase introduced in the present disclosure. The L-tryptophan-producing ability may be imparted or enhanced by improvement of species.

The microorganism of the present disclosure may include all microorganisms into which the pyruvate, phosphate dikinase or a polynucleotide encoding the same is introduced by various known methods.

In one example, the recombinant microorganism having an L-tryptophan-producing ability may include all microorganisms, which may be transformed through a vector and thus capable of producing L-tryptophan by introducing a foreign gene encoding the pyruvate, phosphate dikinase of the present disclosure, specifically, a foreign gene encoding the phosphate dikinase derived from *Komagataeibacter xylinus.*

For example, the microorganism for producing L-tryptophan may be a microorganism into which a polynucleotide sequence encoding a protein including the amino acid sequence of SEQ ID NO: 1, or a protein including an amino acid sequence having at least 60%, 70%, 75%, 80%, 85%, 90%, 95%, 96%, 97%, 98%, 99%, 99.5%, 99.7% or 99.9% or more homology or identity to SEQ ID NO: 1 is introduced.

For example, the microorganism for producing L-tryptophan may be a microorganism into which a polynucleotide capable of encoding a protein including an amino acid sequence having at least 80% homology to the amino acid sequence of SEQ ID NO: 1; or a polynucleotide including a nucleotide sequence of SEQ ID NO: 2 or a nucleotide sequence having 60% or more, 70% or more, 75% or more, 80% or more, 85% or more, 90% or more, 95% or more, 96% or more, 97% or more, 98% or more, or 99% or more homology or identity to the nucleotide sequence of SEQ ID NO: 2 is introduced.

In one example, the microorganism having an increased L-tryptophan-producing ability of the present disclosure may be a microorganism having an increased L-tryptophan-producing ability compared to a non-modified microorganism, but is not limited thereto. In one example, the non-modified microorganism that is the target strain for comparing the increase in the L-tryptophan-producing ability may be the CM05-9157 strain, but is not limited thereto.

In one example, the microorganism having an increased L-tryptophan-producing ability may have an increased L-tryptophan-producing ability by about 1% or more, specifically, about 1% or more, about 2.5% or more, about 5% or more, about 6% or more, about 7% or more, about 8% or more, about 9% or more, about 10% or more, about 15% or more, about 16% or more, about 17%or more, or about 18% or more (the upper limit is not particularly limited, for example, about 200% or less, about 150% or less, about 100% or less, about 50% or less, about 45% or less, about 40% or less, about 35% or less, about 30% or less, about 25% or less, or about 20% or less), as compared to the L-tryptophan-producing ability of a parent microorganism (parent strain) before modification or a non-modified microorganism, but is not limited thereto, as long as it has an increased + value compared to the production ability of a parent microorganism (parent strain) before modification or a non-modified microorganism. In another example, the microorganism having an increased L-tryptophan-producing ability may have an increased L-tryptophan-producing ability by about 1.1 times or more, about 1.15 times or more, about 1.16 times or more, about 1.17 times or more, or about 1.18 times or more (the upper limit is not particularly limited, for example, about 10 times or less, about 5 times or less, about 3 times or less, about 2 times or less, about 1.5 times or less, about 1.4 times or less, about 1.3 times or less, or about 1.2 times or less), as compared to that of a parent microorganism (parent strain) before modification or a non-modified microorganism, but is not limited thereto.

In one example, the microorganism having an L-tryptophan-producing ability may be a prokaryotic cell or eukaryotic cell, but may specifically be a prokaryotic cell. The prokaryotic cell may include, for example, a microorganism belonging to the genus *Escherichia,* the genus *Erwinia,* the genus *Serratia,* the genus *Providencia,* the genus *Corynebacterium,* the genus *Pseudomonas,* the genus *Leptospira,* the genus *Salmonella,* the genus *Brevibacteria,* the genus *Hypomononas,* the genus *Chromobacterium,* and the genus *Norcardia,* or fungi or yeasts, but is not limited thereto. Specifically, it may be a microorganism belonging to the genus *Escherichia,* the genus *Corynebacterium,* the genus *Leptospira,* and yeasts. More specifically, it may be a microorganism belonging to the genus *Corynebacterium.*

As the microorganism according to any one of the above-described embodiments, the microorganism of the present disclosure may be a microorganism of the genus *Corynebacterium.*

In one example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the microorganism of the present disclosure may be a microorganism of the genus *Corynebacterium,* more specifically, *Corynebacterium glutamicum,* but is not limited thereto.

Meanwhile, the microorganism of the genus *Corynebacterium* having an L-tryptophan-producing ability of the present disclosure may include all of the following: a natural wild-type microorganism itself; a microorganism of the genus *Corynebacterium* in which the activity of a gene associated with the mechanism of L-tryptophan production is increased or decreased, thus having an enhanced L-tryptophan-producing ability; or a microorganism of the genus *Corynebacterium* in which the activity of a foreign gene is introduced or increased, thus having an enhanced L-tryptophan-producing ability.

Another aspect of the present disclosure provides a method for producing L-tryptophan, including culturing the microorganism of the genus *Corynebacterium* having an L-tryptophan-producing ability, into which pyruvate, phosphate dikinase derived from *Komagataeibacter xylinus* or a polynucleotide encoding the same is introduced, in a medium.

In the method of the present disclosure, the cultivation of the microorganism may be performed using any culture conditions and cultivation methods known in the art. Such a cultivation process may be easily adjusted for use by those skilled in the art according to the microorganism to be selected.

The L-tryptophan produced by the cultivation of the present disclosure may be released into the medium or remain in the cells.

In one embodiment, the method for producing L-tryptophan of the present disclosure may further include a step of preparing the microorganism of the present disclosure, a step of preparing a medium for culturing the strain, or a combination thereof (regardless of the order, in any order), for example, prior to the culturing step.

The method for producing L-tryptophan of the present disclosure may further include a step of recovering the desired substance, specifically, L-tryptophan, from the cultured microorganism, a culture product of the microorganism, a fermented product of the microorganism, or the culture medium. The recovering step may be further included after the culturing step.

In the recovering step, the desired L-tryptophan may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired substance, specifically, L-tryptophan, can be recovered from the medium or the microorganisms using suitable methods known in the art.

Further, the method for producing L-tryptophan of the present disclosure may further include a purification step, which may be performed using an appropriate method known in the art. In one example, when the method for producing L-tryptophan of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the pyruvate, phosphate dikinase, introduction, and L-tryptophan *etc.* are as described in other aspects above.

Still another aspect of the present disclosure provides a composition for producing L-tryptophan, including: the microorganism of the genus *Corynebacterium* having an L-tryptophan-producing ability, into which pyruvate, phosphate dikinase derived from *Komagataeibacter xylinus* or a polynucleotide encoding the same is introduced; a culture product of the microorganism; a fermented product of the microorganism; or a combination of two or more thereof.

The composition of the present disclosure may further include any suitable excipient commonly used in compositions for producing L-tryptophan, and such excipients may include, for example, preservatives, wetting agents, dispersing agents, suspending agents, buffers, stabilizers, or isotonic agents, *etc.,* but are not limited thereto.

In one embodiment, each component present in the composition of the present disclosure may be contained in a microbiologically effective amount, or in an amount that can be appropriately present in the composition for production.

In the composition of the present disclosure, the pyruvate, phosphate dikinase, introduction, and L-tryptophan *etc.* are as described in other aspects above.

Yet another aspect of the present disclosure provides the use of the microorganism of the genus *Corynebacterium* having an L-tryptophan-producing ability, into which pyruvate, phosphate dikinase derived from *Komagataeibacter xylinus* or a polynucleotide encoding the same is introduced, in the production of L-tryptophan.

In the use of the present disclosure, the pyruvate, phosphate dikinase, introduction, and L-tryptophan *etc.* are as described in other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Screening and Selection of Phosphoenolpyruvate Synthase (ppsA) and Pyruvate, Phosphate Dikinase (ppdK) Genes

In order to select phosphoenolpyruvate synthase or pyruvate and pyruvate, phosphate dikinase with high gluconeogenesis activity, organisms derived from acetic acid bacteria, methanogens, which use carbon sources consisting of 1 to 3 carbons were searched based on the results of a literature search. Among them, four types of microorganisms expected to possess phosphoenolpyruvate synthase or pyruvate and pyruvate, phosphate dikinase were selected, as shown in Table 1 below, in consideration of biosafety levels, which are applicable to the producing strains, and availability.

**[Table 1]**

| Orde r | Strain | Protein Registration No. | Genome Registration No. | Biosafe ty Level |
|---|---|---|---|---|
| 1 | Komagataeibacter xylinus | WP_007399515 .1 | NZ_CP024644 .1 | 1 |
| 2 | Acetobacter pasteurianus subsp. | ASC06387.1 | NZ_CP021922 .1 | 1 |
| 3 | Methanosarcina acetivorans str. C2A | WP_011023333 .1 | NZ_AE010299 .1 | 1 |
| 4 | Escherichia coli | WP_000069375 .1 | NZ_CP084899 .1 | 1 |
| 5 | Corynebacterium glutamicum ATCC13869 | WP_011013725 .1 | NZ_CP016335 .1 | 1 |

### Example 2. Preparation of L-tryptophan-Producing Microorganisms Introduced with Foreign Pyruvate, Phosphate Dikinase

### Example 2-1. Preparation of Plasmid for Inserting Genes

A plasmid was prepared to introduce genes for pyruvate, phosphate dikinase or phosphoenolpyruvate synthase into the transposon gene region within the chromosome of *Corynebacterium glutamicum* by homologous recombination.

Specifically, PCR was performed based on the *Corynebacterium glutamicum* ATCC13869 chromosomal DNA as a template, using a primer pair of SEQ ID NOS: 11 and 12 and a primer pair of SEQ ID NOS: 13 and 14 to obtain respective fragments. Solg^{™} Pfu-X DNA polymerase was used for the PCR, and the PCR was performed under conditions of denaturation at 95°C for 4 minutes, followed by 27 cycles of denaturation at 95°C for 30 seconds, annealing at 60°C for 30 seconds, and polymerization at 72°C for 50 seconds, and then polymerization at 72°C for 5 minutes. The primer sequences used are shown in Table 2.

**[Table 2]**

| SEQ ID NO: | Name | Sequence (5'→ 3') |
|---|---|---|
| SEQ ID NO: 11 | HR1 F | AATTCGAGCTCGGTACCCGATGGAACTACGAGA CTG |
| SEQ ID NO: 12 | HR1 R | TGACAATCACCGCATCCagtactGGATATTCGAGA CAGCTT |
| SEQ ID NO: 13 | HR2 F | GCTGTCTCGAATATCCagtactGGATGCGGTGATT GTCAG |
| SEQ ID NO: 14 | HR2 R | GGTCGACTCTAGAGGATCCCCTAACCACGACGA CTGCAA |

The two fragments amplified by the PCR and the pDCM2 vector (Korean Patent No. 10-2278000) for transformation cleaved by Smal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn.

### Example 2-2. Preparation of Microorganism of the Genus Corynebacterium Introduced with Pyruvate, Phosphate Dikinase derived from Komagataeibacter Xylinus

In order to introduce the *ppdK* gene (NZ_ CP024644.1, SEQ ID NO: 2) encoding pyruvate, phosphate dikinase derived from *Komagataeibacter xylinus (K.xylinus)* into *Corynebacterium glutamicum,* first, PCR was performed based on the *ppdK* gene of *K.xylinus* (SEQ ID NO: 2) synthesized using the Gene-Synthesis service by Bionix Co., Ltd. as a template, using a primer pair of SEQ ID NOS: 15 and 16 to amplify the *ppdK* gene.

Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

**[Table 3]**

| SEQ ID NO: | Name | Sequence (5'→ 3') |
|---|---|---|
| SEQ ID NO: 15 | ppdK(K.xy)-F | cgaaaggaaacactcATGACCAAATGGGTT TACAGC |
| SEQ ID NO: 16 | ppdK(K.xy)-R | AATCACCGCATCCagtTCAGGCCGGGC TGGC |

Additionally, in order to obtain the Pcj7 promoter, PCR was performed based on the p117-cj7-gfp (US 7662943 B2) as a template using primers of SEQ ID NO: 17 and SEQ ID NO: 18. Solg^{™} Pfu-X DNA polymerase (SolGent co.) was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 30 seconds, and then polymerization at 72°C for 5 minutes.

**[Table 4]**

| SEQ ID NO: | Name | Sequence (5'→ 3') |
|---|---|---|
| SEQ ID NO: 17 | Pcj7-F | TCTCGAATATCCagtagaaacatcccagcg ctact |
| SEQ ID NO: 18 | Pci7(K.xy)-R | AACCCATTTGGTCATgagtgtttcctttcgttg gg |

Subsequently, the thus-amplified *ppdK* gene of *K.xylinus,* the Pcj7 promoter region, and the pDCM2-△Tn prepared in Example 2-1 cleaved by Scal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn:: Pcj7-ppdK(K.xy). The cloning was performed by mixing the Gibson assembly reagent and each of the gene fragments in a calculated number of moles, followed by incubating at 50° C. for 1 hour.

The thus-prepared pDCM2-△Tn:: Pcj7-ppdK(K.xy) vector was transformed into the CM05-9157 strain (Korean Patent No. 10-2278000), which is a tryptophan-producing strain, by electroporation *(*Appl. Microbiol. Biotechnol. (1999) 52:541-545) and then subjected to a secondary crossover to obtain a strain, into which a single copy of Pcj7-ppdK(K.xy) gene was inserted between the transposon genes on the chromosome. The resulting strain was identified through genome sequencing and a PCR method using primers of SEQ ID NO: 19 and SEQ ID NO: 20, which can respectively amplify the external region of the upstream region and downstream region of the position where the gene was inserted.

**[Table 5]**

| SEQ ID NO: | Name | Sequence (5'→ 3') |
|---|---|---|
| SEQ ID NO: 19 | Confirm-Pcj7-ppsA/ppdK-F | AACAACACCACATCTA CATC |
| SEQ ID NO: 20 | Confirm-Pcj7-ppsA/ppdK-R | CAGCCTTTTCCAGCAC CA |

The thus-obtained strain was named CM05-9157 :: Pcj7-ppdK(K.xy).

### Example 2-3. Preparation of Microorganism of the Genus Corynebacterium Introduced with Pyruvate, Phosphate Dikinase derived from Acetobacter pasteurianus subsp.

In order to introduce the gene (NZ_CP021922.1, SEQ ID NO: 4) encoding pyruvate, phosphate dikinase derived from *Acetobacter pasteurianus subsp.* (SEQ ID NO: 3) into *Corynebacterium glutamicum,* first, PCR was performed based on the *ppdK* gene of *Acetobacter pasteurianus subsp.* (SEQ ID NO: 4) synthesized using the Gene-Synthesis service by Bionix Co., Ltd. as a template, using a primer pair of SEQ ID NOS: 21 and 22 to amplify the *ppdK* gene.

Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

**[Table 6]**

| SEQ ID NO: | Name | Sequence (5'→ 3') |
|---|---|---|
| SEQ ID NO: 21 | ppdK(A.pa)-F | cgaaaggaaacactcATGCGCAATCTCCTCGG C |
| SEQ ID NO: 22 | ppdK(A.pa)-R | GCTGACAATCACCGCATCCagtTCAGGCTT TTACGGCCTTG |

Additionally, in order to obtain the Pcj7 promoter, PCR was performed based on the p117-cj7-gfp (US 7662943 B2) as a template using primers of SEQ ID NO: 17 and SEQ ID NO: 23. Solg^{™} Pfu-X DNA polymerase (SolGent co.) was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 30 seconds, and then polymerization at 72°C for 5 minutes.

**[Table 7]**

| SEQ ID NO: | Name | Sequence (5'→ 3') |
|---|---|---|
| SEQ ID NO: 23 | Pcj7(A.pa)-R | GAGGAGATTGCGCATgagtgtttcctttcgtt ggg |

Subsequently, the thus-amplified *ppdK* gene of *Acetobacter pasteurianus subsp.,* the Pcj7 promoter region, and the pDCM2-△Tn prepared in Example 2-1 cleaved by Scal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn:: Pcj7-ppdK(A.pa). The cloning was performed by mixing the Gibson assembly reagent and each of the gene fragments in a calculated number of moles, followed by incubating at 50°C. for 1 hour.

Thereafter, the thus-prepared pDCM2-△Tn:: Pcj7-ppdK(A.pa) vector was transformed into the CM05-9157 strain (Korean Patent No. 10-2278000), which is a tryptophan-producing strain, by electroporation *(*Appl. Microbiol. Biotechnol. (1999) 52:541-545) and then subjected to a secondary crossover to obtain a strain, into which a single copy of Pcj7-ppdK(A.pa) gene was inserted between the transposon genes on the chromosome. The resulting strain was identified through genome sequencing and a PCR method using primers of SEQ ID NO: 19 and SEQ ID NO: 20, which can respectively amplify the external region of the upstream region and downstream region of the position where the gene was inserted.

The thus-obtained strain was named CM05-9157 :: Pcj7-ppdK(A.pa).

### Example 2-4. Preparation of Microorganism of the Genus Corynebacterium Introduced with Pyruvate, Phosphate Dikinase derived from Methanosarcina acetivorans str. C2A

In order to introduce the gene (NZ_AE010299.1, SEQ ID NO: 6) encoding pyruvate, phosphate dikinase derived from *Methanosarcina acetivorans str. C2A* (SEQ ID NO: 5) into *Corynebacterium glutamicum,* first, PCR was performed based on the *ppdK* gene of *Methanosarcina acetivorans str. C2A* (SEQ ID NO: 6) synthesized using the Gene-Synthesis service by Bionix Co., Ltd. as a template, using a primer pair of SEQ ID NOS: 24 and 25 to amplify the *ppdK* gene.

Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

**[Table 8]**

| SEQ ID NO: | Name | Sequence (5'→ 3') |
|---|---|---|
| SEQ ID NO: 24 | ppdK(M.ac)-F | acgaaaggaaacactcATGCCTGGAGATAAAAA CAAATACATC |
| SEQ ID NO: 25 | ppdK(M.ac)-R | AATCACCGCATCCagtTCAGAGCTCCTTTTC AGTTTC |

Additionally, in order to obtain the Pcj7 promoter, PCR was performed based on the p117-cj7-gfp (US 7662943 B2) as a template using primers of SEQ ID NO: 17 and SEQ ID NO: 26. Solg^{™} Pfu-X DNA polymerase (SolGent co.) was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 30 seconds, and then polymerization at 72°C for 5 minutes.

**[Table 9]**

| SEQ ID NO: | Name | Sequence (5'→ 3') |
|---|---|---|
| SEQ ID NO: 26 | Pcj7(M.ac)-R | TTTATCTCCAGGCATgagtgtttcctttcgtt ggg |

Subsequently, the thus-amplified *ppdK* gene of *Methanosarcina acetivorans str. C2A.,* the Pcj7 promoter region, and the pDCM2-△Tn prepared in Example 2-1 cleaved by Scal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn:: Pcj7-ppdK(M.ac). The cloning was performed by mixing the Gibson assembly reagent and each of the gene fragments in a calculated number of moles, followed by incubating at 50° C. for 1 hour.

Thereafter, the thus-prepared pDCM2-△Tn:: Pcj7-ppdK(M.ac) vector was transformed into the CM05-9157 strain (Korean Patent No. 10-2278000), which is a tryptophan-producing strain, by electroporation *(*Appl. Microbiol. Biotechnol. (1999) 52:541-545) and then subjected to a secondary crossover to obtain a strain, into which a single copy of Pcj7-ppdK(M.ac) gene was inserted between the transposon genes on the chromosome. The resulting strain was identified through genome sequencing and a PCR method using primers of SEQ ID NO: 19 and SEQ ID NO: 20, which can respectively amplify the external region of the upstream region and downstream region of the position where the gene was inserted.

The thus-obtained strain was named CM05-9157 :: Pcj7-ppdK(M.ac).

### Example 2-5. Preparation of Microorganism of the Genus Corynebacterium Introduced with Pyruvate, Phosphate Dikinase derived from Escherichia coli

In order to introduce the *ppsA* (NZ_CP084899.1, SEQ ID NO: 8) encoding phosphoenolpyruvate synthase derived from *Escherichia coli* (SEQ ID NO: 7), which is an ortholog of the *ppdk* gene, into *Corynebacterium glutamicum,* first, PCR was performed based on the *Escherichia coli* W3110 chromosomal DNA as a template, using a primer pair of SEQ ID NOS: 27 and 28 to amplify the *ppsA* gene.

Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

**[Table 10]**

| SEQ ID NO: | Name | Sequence (5'→ 3') |
|---|---|---|
| SEQ ID NO: 27 | ppsA(E.co)-F | aacgaaaggaaacactcATGTCCAACAATGGC TCG |
| SEQ ID NO: 28 | ppsA(E.co)-R | AATCACCGCATCCagtTTATTTCTTCAGTT CAGCCAGG |

Additionally, in order to obtain the Pcj7 promoter, PCR was performed based on the p117-cj7-gfp (US 7662943 B2) as a template using primers of SEQ ID NO: 17 and SEQ ID NO: 29. Solg^{™} Pfu-X DNA polymerase (SolGent co.) was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 30 seconds, and then polymerization at 72°C for 5 minutes.

**[Table 11]**

| SEQ ID NO: | Name | Sequence (5'→ 3') |
|---|---|---|
| SEQ ID NO: 29 | Pcj7(E.co)-R | GCCATTGTTGGACATgagtgtttcctttcgttg gg |

Subsequently, the thus-amplified *ppsA* gene of *Escherichia coli,* the Pcj7 promoter region, and the pDCM2-△Tn prepared in Example 2-1 cleaved by Scal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn:: Pcj7-ppsA(E.co). The cloning was performed by mixing the Gibson assembly reagent and each of the gene fragments in a calculated number of moles, followed by incubating at 50° C. for 1 hour.

Thereafter, the thus-prepared pDCM2-△Tn:: Pcj7-ppsA(E.co) vector was transformed into the CM05-9157 strain (Korean Patent No. 10-2278000), which is a tryptophan-producing strain, by electroporation *(*Appl. Microbiol. Biotechnol. (1999) 52:541-545) and then subjected to a secondary crossover to obtain a strain, into which a single copy of Pcj7-ppsA(E.co) gene was inserted between the transposon genes on the chromosome. The resulting strain was identified through genome sequencing and a PCR method using primers of SEQ ID NO: 19 and SEQ ID NO: 20, which can respectively amplify the external region of the upstream region and downstream region of the position where the gene was inserted.

The thus-obtained strain was named CM05-9157 :: Pcj7-ppsA(E.co).

### Example 2-6. Preparation of Microorganism of the Genus Corynebacterium Introduced with Pyruvate, Phosphate Dikinase derived from Corynebacterium glutamicum ATCC13869

In order to introduce the *ppsA* (NZ_CP016335.1, SEQ ID NO: 10) encoding phosphoenolpyruvate synthase derived from *Corynebacterium glutamicum* ATCC13869 (SEQ ID NO: 9), which is an ortholog of the *ppdk gene,* into *Corynebacterium glutamicum,* first, PCR was performed based on the *Corynebacterium glutamicum* ATCC13869 chromosomal DNA as a template, using a primer pair of SEQ ID NOS: 30 and 31 to amplify the *ppsA* gene.

Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

**[Table 12]**

| SEQ ID NO: | Name | Sequence (5'→ 3') |
|---|---|---|
| SEQ ID NO: 30 | ppsA(C.gl)-F | caacgaaaggaaacactcATGACCAACAGTTT GAACATCC |
| SEQ ID NO: 31 | ppsA(C.gl)-R | ACAATCACCGCATCCagtTTACTTCGTG CCGGTCATTG |

Additionally, in order to obtain the Pcj7 promoter, PCR was performed based on the p117-cj7-gfp (US 7662943 B2) as a template using primers of SEQ ID NO: 17 and SEQ ID NO: 32. Solg^{™} Pfu-X DNA polymerase (SolGent co.) was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 30 seconds, and then polymerization at 72°C for 5 minutes.

**[Table 13]**

| SEQ ID NO: | Name | Sequence (5'→ 3') |
|---|---|---|
| SEQ ID NO: 32 | Pci7(C.gl)-R | CAAACTGTTGGTCATgagtgtttcctttcgttgg g |

Subsequently, the thus-amplified *ppsA* gene of *Corynebacterium glutamicum* ATCC13869, the Pcj7 promoter region, and the pDCM2-△Tn prepared in Example 2-1 cleaved by Scal restriction enzyme were cloned using the Gibson assembly method (DG Gibson et al., NATURE METHODS, VOL. 6 NO. 5, May 2009, NEBuilder HiFi DNA Assembly Master Mix) to obtain a recombinant plasmid, which was named pDCM2-△Tn:: Pcj7-ppsA(C.gl). The cloning was performed by mixing the Gibson assembly reagent and each of the gene fragments in a calculated number of moles, followed by incubating at 50° C. for 1 hour.

Thereafter, the thus-prepared pDCM2-△Tn:: Pcj7-ppsA(C.gl) vector was transformed into the CM05-9157 strain (Korean Patent No. 10-2278000), which is a tryptophan-producing strain, by electroporation *(*Appl. Microbiol. Biotechnol. (1999) 52:541-545) and then subjected to a secondary crossover to obtain a strain, into which a single copy of Pcj7-ppsA(C.gl) gene was inserted between the transposon genes on the chromosome. The resulting strain was identified through genome sequencing and a PCR method using primers of SEQ ID NO: 19 and SEQ ID NO: 20, which can respectively amplify the external region of the upstream region and downstream region of the position where the gene was inserted.

The thus-obtained strain was named CM05-9157 :: Pcj7-ppsA(C.gl).

### Example 3. Evaluation of L-Tryptophan-Producing Ability of L-Tryptophan-Producing Microorganisms Introduced with Foreign Pyruvate, Phosphate Dikinase or Ortholog Thereof

In order to confirm the L-Tryptophan-producing ability of the CM05-9157 :: Pcj7-ppdK(K.xy), CM05-9157 :: Pcj7-ppdK(A.pa), CM05-9157 :: Pcj7-ppdK(M.ac), and CM05-9157 :: Pcj7-ppsA(E.co) strains prepared in Examples 2-2, 2-3, 2-4, and 2-5, respectively, the parent strain CM05-9157, which was not introduced with a foreign gene, and the CM05-9157 :: Pcj7-ppsA(C.gl) prepared in Example 2-6, the strains were cultured and evaluated in the following manner.

Each of the strains was inoculated into a 250-mL corner-baffled flask containing 25 mL of the following seed medium and was cultured with shaking at 200 rpm at 30° C. for 20 hours. Next, 1 mL of the seed culture solution was inoculated into a 250-mL corner-baffled flask containing 25 mL of the following production medium and was cultured with shaking at 200 rpm at 30° C. for 24 hours. After the culture, the production amount of L-tryptophan was measured by HPLC. The compositions of the seed medium and production medium are as follows, and the L-tryptophan concentration in the culture medium for each strain tested is shown in Table 14 below.

### <Seed Medium (pH 7.0)>

glucose 20 g, peptone 10 g, yeast extract 5 g, urea 1.5 g, KH₂PO₄ 4 g, K₂HPO₄ 8 g, MgSO₄ 7H₂O 0.5 g, biotin 100 µg, thiamine HCl 1000 µg, calcium-pantothenate 2000 µg, nicotinamide 2000 µg (per liter of distilled water).

### <Production Medium (pH 7.0)>

glucose 30g, (NH₄)₂SO₄ 15 g, MgSO₄ 7H₂O 1.2 g, KH₂PO₄ 1 g, yeast extract 5 g, biotin 900 µg, thiamine HCl 4500 µg, calcium-pantothenate 4500 µg, CaCO₃ 30 g (per liter of distilled water).

**[Table 14]**

| | | | OD562 | Amount of Tryptophan Production (g/L) | Tryptophan Yield (*100 g/g, %) |
|---|---|---|---|---|---|
| CM05-9157 | | | 56.5 | 1.88 | 6.37 |
| CM05-9157 ppsA(C.gl) | :: | Pcj7- | 56.3 | 1.89 | 6.38 |
| CM05-9157 Pcj7ppsA(E.co) | | :: | 56.1 | 1.9 | 6.40 |
| CM05-9157 ppdK(A.pa) | :: | Pcj7- | 56.8 | 1.85 | 6.34 |
| CM05-9157 ppdK(K.xy) | :: | Pcj7- | 52.1 | 2.35 | 7.52 |
| CM05-9157 ppdK(M.ac) | :: | Pcj7- | 57.8 | 1.64 | 5.51 |

As a result, as shown in Table 14 above, it was confirmed that the amount of tryptophan production of the CM05-9157 :: Pcj7-ppsA(C.gl) strain introduced with the phosphoenolpyruvate synthase gene derived from *Corynebacterium glutamicum* was 1.89 g/L, which was almost the same as the amount of tryptophan production of the parent strain, CM05-9157 strain.

Additionally, among the pyruvate, phosphate dikinase genes or the phosphoenolpyruvate synthase genes, which are orthologs thereof, derived from various microorganisms, it was confirmed that only the CM05-9157 :: Pcj7-ppdK(K.xy) strain introduced with the pyruvate, phosphate dikinase gene derived from *Komagataeibacter xylinus* produced a final 2.35 g/L of L-tryptophan in the flask culture, increasing the L-tryptophan-producing ability by about 18% compared to the parent strain, CM05-9157, and/or the CM05-9157 :: Pcj7-ppsA(C.gl) strain.

In contrast, it was confirmed that the three types of strains (CM05-9157 :: Pcj7-ppdK(A.pa), CM05-9157 :: Pcj7-ppdK(M.ac), and CM05-9157 :: Pcj7-ppsA(E.co)) introduced with the foreign phosphoenolpyruvate synthase or pyruvate, phosphate dikinase derived from other than *Komagataeibacter xylinus* showed a slight decrease in the tryptophan production compared to the control group, CM05-9157, or the increase in the tryptophan production was relatively insignificant.

These results imply that the L-tryptophan-producing ability could only be specifically increased when the particular pyruvate, phosphate dikinase genes derived from *Komagataeibacter xylinus* were introduced into the microorganism of the genus *Corynebacterium.*

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A microorganism of the genus *Corynebacterium* having an L-tryptophan-producing ability, into which pyruvate, phosphate dikinase derived from *Komagataeibacter xylinus* or a polynucleotide encoding the same is introduced.

2. The microorganism of claim 1, wherein the pyruvate, phosphate dikinase derived from *Komagataeibacter xylinus* comprises an amino acid sequence of SEQ ID NO: 1 or an amino acid sequence having at least 90% identity with SEQ ID NO: 1.

3. The microorganism of claim 1, wherein the pyruvate, phosphate dikinase derived from *Komagataeibacter xylinus* is encoded by a *ppdK* gene.

4. The microorganism of claim 1, wherein the microorganism of the genus *Corynebacterium* is *Corynebacterium glutamicum.*

5. The microorganism of any one of claims 1 to 4, wherein the microorganism of the genus *Corynebacterium* has an increased L-tryptophan-producing ability compared to a non-modified microorganism.

6. A method for producing L-tryptophan, comprising culturing a microorganism of the genus *Corynebacterium* having an L-tryptophan-producing ability, into which pyruvate, phosphate dikinase derived from *Komagataeibacter xylinus* or a polynucleotide encoding the same is introduced, in a medium.

7. The method of claim 6, further comprising recovering L-tryptophan from the cultured microorganism, a culture product of the microorganism, a fermented product of the microorganism, or the culture medium.

8. A composition for producing L-tryptophan, comprising: a microorganism of the genus *Corynebacterium* having an L-tryptophan-producing ability, into which pyruvate, phosphate dikinase derived from *Komagataeibacter xylinus* or a polynucleotide encoding the same is introduced; a culture product of the microorganism; a fermented product of the microorganism; or a combination of two or more thereof.

9. Use of a composition, which comprises a microorganism of the genus *Corynebacterium* having an L-tryptophan-producing ability, into which pyruvate, phosphate dikinase derived from *Komagataeibacter xylinus* or a polynucleotide encoding the same is introduced; a culture product of the microorganism; a fermented product of the microorganism; or a combination of two or more thereof, for the production of L-tryptophan.
